# EUROPÄISCHE PATENTSCHRIFT

(11) **EP 4 027 957 B1**
(45) Veröffentlichungstag und Bekanntmachung des Hinweises auf die Patenterteilung: **22.01.2025**
(21) Anmeldenummer: 20768034.9
(22) Anmeldetag: 08.09.2020
(51) Int. Cl.: A61F 9/008

(54) **POSITIONIEREINRICHTUNG**
POSITIONING DEVICE
DISPOSITIF DE POSITIONNEMENT

(30) Priorität: 10.09.2019 DE 102019213698; 06.12.2019 DE 102019219122
(43) Veröffentlichungstag der Anmeldung: 20.07.2022
(73) Patentinhaber: Carl Zeiss Meditec AG, 07745 Jena (DE)
(72) Erfinder: BÖHME, Beate, 07751 Großpürschütz (DE); STOBRAWA, Gregor, 07743 Jena (DE); FESTAG, Karsten, 07749 Jena (DE); LEHNORT, Marco, 07751 Jena (DE)
(74) Vertreter: Nieten, Christoph
(86) Internationale Anmeldenummer: PCT/EP2020/075035
(87) Internationale Veröffentlichungsnummer: WO 2021/048100

(56) Entgegenhaltungen:
- EP-A1- 1 223 847
- EP-B1- 1 223 847
- WO-A1-2010/000279
- DE-A1- 102006 053 580
- US-A1- 2006 192 921

## Beschreibung

Die vorliegende Erfindung betrifft eine Positioniereinrichtung für ein ophthalmologisches Lasertherapiesystem, das eine optische Öffnung aufweist, zur Positionierung eines Auges gegenüber der optischen Öffnung. Die Erfindung betrifft weiterhin ein ophthalmologisches Lasertherapiesystem. Die Erfindung betrifft schließlich ein Verfahren für eine Positioniereinrichtung eines ophthalmologischen Lasertherapiesystems, das eine optische Öffnung aufweist, zur Positionierung eines Auges eines Patienten gegenüber der optischen Öffnung.

In der ophthalmologischen Lasertherapie ist es mittlerweile üblich, eine Lasertherapievorrichtung zur Erzeugung von Schnitten in einem Augengewebe, oder zur Ablation oder zur Koagulation eines Augengewebes mittels Laserstrahlung zu kombinieren mit einer Beobachtungseinrichtung zur Kontrolle und Überwachung verschiedener Arbeitsschritte während des therapeutischen Eingriffs.

Dies ist beispielsweise sehr vorteilhaft in der laserunterstützten Augenchirurgie zur Korrektur von Fehlsichtigkeit oder zur Therapie von anderen Augenerkrankungen wie z.B. einem Katarakt mittels der Kataraktchirurgie. Auch bei Fehlsichtigkeitskorrekturen wie beispielsweise einer "SMILE"-Behandlung, also einer Lentikelextraktion durch kleine Inzision ("Small Incision Lenticule Extraction"), einer Implantation eines Lentikels oder bei anderen Modifikationen der Hornhaut ist eine Überwachung der Arbeitsschritte notwendig, um das Anbringen der Schnitte in der Cornea gegebenenfalls abbrechen zu können. Nach dem Anbringen der Schnitte im Augengewebe wird unter Beobachtung - beispielsweise durch ein Operationsmikroskop (auch OPMI genannt) - das Lentikel entnommen (oder modifiziert) oder ein Implantat eingeführt.

Ein wichtiger Arbeitsschritt bei der Vorbereitung einer ophthalmologischen Lasertherapie (insbesondere bei der Anwendung von fs-Lasern) ist eine Ausrichtung des Lasertherapiesystems gegenüber dem Auge oder sogar eine Augenfixation. Häufig erfolgt dies unter Verwendung eines Kontaktelementes (z.B. eines Kontaktglases), das der Bediener (Chirurg, Assistent oder allgemein Benutzer) des ophthalmologischen Lasertherapiesystems mit dem Patientenauge in Kontakt bringen muss. Danach wird das Auge gegenüber dem Lasertherapiesystem immobilisiert. Gegebenenfalls sind in Abhängigkeit von der relativen Lage des immobilisierten Auges zum Lasertherapiesystem Geräteparameter zu justieren. Erst anschließend kann der Benutzer den Therapieprozess starten, dessen Fortgang er in der Regel überwachen muss.

In DE102005013949 ist ein ophthalmologisches Lasertherapiesystem zur Behandlung des menschlichen Auges beschrieben, das eine Strahlquelle und Vorrichtungen zum dreidimensionalem (x,y,z) Scannen des fokussierten Laserstrahles am Auge aufweist. Auf der Cornea des Auges befindet sich dabei ein Kontaktglas, das an der Cornea des Auges anliegt. Im Strahlengang des Lasertherapiesystems ist ein Strahlteiler angeordnet, mit dem vom Auge ausgehende Strahlenbündel wenigstens teilweise in Richtung auf einen Tubus mit Okular (zur direkten Betrachtung durch den Bediener) oder ein Objektiv mit Kamera abgelenkt werden. Dies erlaubt eine Mitbeobachtung der Cornea, während das Auge mit Licht eines fs-Laser-beaufschlagt wird (Therapie). Die beschriebene Anordnung erlaubt zwar die Überwachung der Therapie, eine Kontrolle des Positionierens des Lasertherapiesystems gegenüber dem Patientenauge sowie des Andockens des Auges an das Kontaktglas wird nur durch diese zum Therapiestrahlengang koaxial angeordnete Mitbeobachtung bereitgestellt.

In WO 2016/058931 ist ein ophthalmologisches Lasertherapiesystem beschrieben, das ein Laser-System (für die Therapie) und ein Operationsmikroskop (zur Untersuchung) aufweist, die miteinander verbunden werden können. Auch die hier offenbarte Anordnung erlaubt die Überwachung der Therapie, eine Kontrolle der Positionierung des Auges wird nicht bereitgestellt.

Auch weitere Lasertherapiesysteme, wie sie in DE 102005032946A1 und US 10123696B2 beschrieben sind, erlauben lediglich eine Beobachtung (und Überwachung) des Auges mit einem Strahlengang, der koaxial zum Therapiestrahlengang verläuft. So wird in DE 102005032946A1 in beide Teile eines binokularen Strahlenganges des Operationsmikroskops das gleiche Bild eingekoppelt; dabei verläuft die Beobachtungsrichtung koaxial zum Therapiestrahlengang. In WO 2019/068866 ist ein Kamerasystem offenbart, das unter einem Beobachtungswinkel zum Therapiestrahlengang auf das Auge gerichtet ist, und das ebenfalls nur zur Beobachtung während der Therapie (oder unmittelbar vor dem Anliegen des Auges am Kontaktelement) genutzt werden kann.

Alle diese Systeme sind nicht dazu geeignet, eine Positionierung des Auges gegenüber dem ophthalmologischen Lasertherapiesystem zu ermöglichen. Weitere Positioniersysteme sind aus den Dokumenten US2006/192921, WO2010/000279 und DE102006053580 bekannt.

Aufgabe der vorliegenden Erfindung ist es deshalb, die Nachteile des Standes der Technik zu beseitigen und eine Lösung für eine Positioniereinrichtung für ein ophthalmologisches Lasertherapiesystem zu Verfügung zu stellen, die eine genaue, schnelle, benutzerfreundliche und intuitive Positionierung des Lasertherapiesystems gegenüber dem Patientenauge erlaubt. Erfindungsgemäß wird die Aufgabe durch die Merkmale der unabhängigen Ansprüche gelöst. Bevorzugte Weiterbildungen und Ausgestaltungen sind Gegenstand der abhängigen Ansprüche.

Ein erster Aspekt der Erfindung betrifft eine Positioniereinrichtung für ein ophthalmologisches Lasertherapiesystem, das eine optische Öffnung aufweist, zur Positionierung eines Auges gegenüber der optischen Öffnung.

Ein ophthalmologisches Lasertherapiesystem ist dazu ausgebildet, bei einer Verwendung durch einen Benutzer (Operateur, Chirurgen, Bediener) eine ophthalmologische Therapie durchzuführen. Unter einer ophthalmologischen Therapie soll dabei jegliche Therapie verstanden werden, bei der ein Gewebe eines Auges verändert wird. Die ophthalmologische Therapie beinhaltet insbesondere entsprechende laserchirurgische Eingriffe, bei denen mittels eines Lasers, bevorzugt eines gepulsten Lasers wie beispielsweise eines Femtosekunden- oder Excimer-Lasers, durch Photodisruption ein Gewebe des Auges "geschnitten" wird (z.B. eines Lentikels oder eines Flaps), durch eine Ablationswirkung ein Bereich eines Augengewebes abgetragen wird oder durch eine Koagulationswirkung Augengewebe miteinander "verklebt" wird bzw. der Brechungsindex des Materials, also eines Augengewebes oder aber eines Implantats, durch die Laserstrahlung verändert wird.

Über die optische Öffnung des ophthalmologischen Lasertherapiesystems kann beispielsweise das Licht einer Laserquelle aus dem Lasertherapiesystem austreten und - bei Positionierung des Auges in einer Zielposition oder nahe der Zielposition - in das Auge des Patienten eindringen, um dort die geplante Therapie durchzuführen. Bei der optischen Öffnung kann es sich also um eine Austrittsöffnung für Therapiestrahlung handeln (die Laseraustrittsöffnung). Über die optische Öffnung des ophthalmologischen Lasertherapiesystems kann auch vom Auge reflektiertes oder gestreutes Licht in das Lasertherapiesystem eintreten und - bei geeigneter Positionierung in der oder nahe der Zielposition - somit eine Untersuchung des Auges ermöglichen (bzw. eine Beobachtung des Auges für "manuelle" Operationsschritte wie der Entnahme eines Lentikels). Bei der optischen Öffnung kann es sich also um eine Eintrittsöffnung für Untersuchungsstrahlung handeln.

Die optische Öffnung kann als Kontaktelement oder Kontaktglas ausgeformt sein. Die optische Öffnung kann die Abschlusslinse eines Operationsmikroskops sein.

Erfindungsgemäß weist die Positioniereinrichtung eine erste Aufnahmeeinheit (auch Beobachtungseinheit genannt) auf, die dazu ausgebildet ist, erste Aufnahmedaten (auch Beobachtungsdaten genannt) vom Auge aus einer ersten Aufnahmerichtung (auch Beobachtungswinkeln genannt) bereitzustellen. Weiterhin weist die Positioniereinrichtung eine zweite Aufnahmeeinheit auf, die dazu ausgebildet ist, zweite Aufnahmedaten vom Auge aus einer zweiten Aufnahmerichtung bereitzustellen. Dabei ist die zweite Aufnahmerichtung von der ersten Aufnahmerichtung verschieden. Weiterhin weist die Positioniereinrichtung eine dritte Aufnahmeeinheit, die dazu ausgebildet ist, dritte Aufnahmedaten aus einer dritten Aufnahmerichtung bereitzustellen, wobei die dritte Aufnahmerichtung von der ersten und zweiten Aufnahmerichtung verschieden ist.

Unter der Aufnahmerichtung einer Aufnahmeeinheit ist ein Vektor zu verstehen, der von der Aufnahmeeinheit in die Richtung zeigt, von der Aufnahmedaten aufgenommen werden können; er weist in "Blickrichtung" der Aufnahmeeinheit. In Blickrichtung der Aufnahmeeinheit befindet sich vorzugsweise die Zielposition des Auges. Ein Winkel zwischen den beiden Aufnahmerichtungen beträgt bevorzugt nicht 180°. Die beiden Aufnahmeeinheiten, die beispielsweise als Kameras mit flächigen Sensoren und mit Kameraoptiken (auch Aufnahmeoptik oder Beobachtungsoptiken genannt) ausgestaltet sein können, erlauben somit eine Beobachtung des Auges unter zwei verschiedenen Winkeln.

Eine Aufnahmeeinheit ist bevorzugt klein, d.h. der sichtbare Optik-Durchmesser der Aufnahmeoptik ist kleiner als 10mm, besonders bevorzugt kleiner als 5mm, und/oder die F-Number ist größer als 2. Weiterhin ist der Sensor (z.B. CCD, CMOS) der Aufnahmeeinheit bevorzugt kleiner als 10mm x 8mm, die Sensor-Pixel sind kleiner als 3µm x 3µm und/oder der Sensor weist mindestens 1900x2500 Pixel auf. Bevorzugt weist die Aufnahmeeinheit ein Volumen von weniger als 3cm x 3cm x 3cm auf.

Weiterhin umfasst die Positioniereinrichtung eine Verschiebeeinheit, die dazu ausgebildet ist, die relative Lage des Auges gegenüber der optischen Öffnung auf Basis von Steuerbefehlen zu verschieben. Dabei kann die Verschiebeeinheit beispielsweise dazu ausgebildet sein, die optische Öffnung zu verschieben. Alternativ oder zusätzlich kann die Verschiebeeinheit dazu ausgebildet sein, das Auge zu verschieben - beispielsweise durch eine Verschiebung einer Patientenliege, auf der sich der Patient befindet. Vorzugsweise wird eine Verschiebung in allen drei Raumrichtungen ermöglicht. Richtung und Geschwindigkeit der Verschiebung werden über Steuerbefehle gesteuert. Diese werden erfindungsgemäß von einer Steuereinheit bereitgestellt, die ebenfalls Teil der Positioniereinrichtung ist. Bei der Steuereinheit kann es sich um einen Computer handeln, der einen Prozessor und einen Speicher aufweist. Die Steuereinheit ist dazu ausgebildet, die Steuerbefehle auf Basis der Aufnahmedaten zu erzeugen. Dies kann durch ein Verrechnen der Aufnahmedaten erfolgen (beispielsweise in einer Recheneinheit, die Teil der Steuereinheit ist). Dabei kann über flächige Aufnahmedaten aus den drei verschiedenen Aufnahmerichtungen eine Position des Auges gegenüber dem ophthalmologischen Lasertherapiesystem und dessen optischer Öffnung errechnet werden. Bevorzugt werden bei der Berechnung der dreidimensionalen Position des Auges die relativen Lagen der Aufnahmeeinheiten sowie deren Aufnahmerichtungen (und/oder beispielsweise auch eine Vergrößerung oder eine Fokuslage) berücksichtigt. Bei der Berechnung der relativen Lage zwischen der optischen Öffnung und dem Auge können zusätzlich die relativen Lagen der optischen Öffnung gegenüber den Aufnahmeeinheiten berücksichtigt werden. Aus der ermittelten Position des Auges gegenüber der optischen Öffnung lassen sich die erforderlichen Steuerbefehle für die Verschiebeeinheit ableiten.

Die erfindungsgemäße Positioniereinrichtung erlaubt eine automatische Positionierung des Auges gegenüber der optischen Öffnung des ophthalmologischen Lasertherapiesystems.

Die Positionierung kann auch das Andocken an ein Kontaktglas umfassen. Das Ergebnis der Positionierung kann vom Bediener verifiziert werden; die Verifikation muss dabei nicht über die Positioniereinrichtung erfolgen. Nach der Verifikation kann der Bediener die Positionierung wiederholen oder die Therapie des Auges beginnen.

Die Aufnahmeeinheiten sind bevorzugt fest mit dem ophthalmologischen Lasertherapiesystem verbunden. Weist das ophthalmologische Lasertherapiesystem eine ortsfeste Gerätebasis und einen dagegen beweglichen (beispielsweise lateral in x-y-Richtung und/oder in der Höhe z) Gerätkopf auf, so ist eine Aufnahmeeinheit bevorzugt am Gerätekopf befestigt. Sind am Gerätekopf ein Laserschwenkarm (zur Bereitstellung von Therapiestrahlung) bzw. ein Untersuchungsschwenkarm (zur Untersuchung von Untersuchungsstrahlung) befestigt, die die optische Öffnung aufweisen, die gegenüber der Gerätebasis bewegt werden kann (vorzugsweise in z-Richtung, aber auch in x-y-Richtung), so ist die andere Aufnahmeeinheit bevorzugt an einem der Schwenkarme - besonders bevorzugt am Laserschwenkarm - befestigt. Eine Positionierung am Laserschwenkarm ist deshalb bevorzugt, da eine genaue Positionierung des Auges gegenüber der optischen Öffnung für die Therapie deutlichen kritischer ist als für eine Untersuchung. Weiterhin ist die Aufnahmeoptik bevorzugt nahe der optischen Öffnung angeordnet.

Eine alternative Positioniereinrichtung für ein ophthalmologisches Lasertherapiesystem, das eine optische Öffnung aufweist, zur Positionierung eines Auges gegenüber der optischen Öffnung umfasst eine erste Aufnahmeeinheit, die dazu ausgebildet ist, erste Aufnahmedaten vom Auge aus einer ersten Aufnahmerichtung bereitzustellen. Weiterhin weist sie eine zweite Aufnahmeeinheit auf, die dazu ausgebildet ist, zweite Aufnahmedaten vom Auge aus einer zweiten Aufnahmerichtung bereitzustellen, wobei die zweite Aufnahmerichtung von der ersten Aufnahmerichtung verschieden ist. Weiterhin weist sie eine dritte Aufnahmeeinheit auf, die dazu ausgebildet ist, dritte Aufnahmedaten aus einer dritten Aufnahmerichtung bereitzustellen, wobei die dritte Aufnahmerichtung von der ersten und zweiten Aufnahmerichtung verschieden ist. Darüber hinaus umfasst die Positioniereinrichtung eine Verschiebeeinheit, die dazu ausgebildet ist, die relative Lage des Auges gegenüber der optischen Öffnung auf Basis von Steuerbefehlen zu verschieben. Dabei gelten die oben getroffenen Beschreibungen zu den Aufnahmeeinheiten und der Verschiebeeinheit auch für diese alternative Positioniereinrichtung. Zusätzlich weist die Positioniereinrichtung eine Darstellungseinheit zur Darstellung der ersten und zweiten Aufnahmedaten vom Auge auf. Dazu können die Aufnahmedaten zunächst in Darstellungsdaten umgewandelt werden. Aufgrund der unterschiedlichen Aufnahmerichtungen der Aufnahmeeinheiten wird der Bediener in die Lage versetzt, die dreidimensionale Position des Auges auf der Darstellungseinheit zu erkennen, solange sich das Auge in den Bildfeldern (häufig auch als Objektfelder bezeichnet) der Aufnahmeeinheiten befindet.

Bevorzugt befindet sich die Darstellungseinheit nahe an dem Ort, den der Bediener während der Therapie oder bei der Vorbereitung dazu (z.B. beim Andocken des Auges an das Kontaktglas) einnimmt. Vorteilhaft befindet sich die Darstellungseinheit in Blickrichtung und in einer angenehmen Sehweite zum Bediener. Die Darstellungseinheit kann beispielsweise mit dem Laserschwenkarm oder mit dem Untersuchungsschwenkarm verbunden sein. Weiterhin können dem Bediener auf dieser Darstellungseinheit zusätzliche Informationen oder Menüs zur Steuerung des Lasertherapiesystems dargestellt werden.

Weiterhin umfasst die Positioniereinrichtung eine Eingabeeinheit, die eine Eingabe von Eingabedaten durch den Bediener ermöglicht. Bei der Eingabeeinheit kann es sich beispielsweise um ein oder mehrere Knöpfe, eine Tastatur, Dreh- und/oder Schieberegler handeln. Die Eingabeeinheit kann auch in die Darstellungseinheit integriert sein als Touch-Screen. Bevorzugt ist ein Joystick, der zusätzlich noch einen oder mehrere Knöpfe aufweisen kann.

Darüber hinaus weist die Positioniereinrichtung eine Steuereinheit auf. Diese ist dazu ausgebildet, Steuerbefehle auf Basis der Aufnahmedaten bereitzustellen (entsprechend der oben getroffenen Beschreibungen der Steuereinheit). Zusätzlich oder alternativ ist sie dazu ausgebildet, auf Basis der Eingabedaten der Eingabeeinheit Steuerbefehle zu erzeugen. Die Eingabedaten können also von der Steuereinheit in die Steuerbefehle für die Verschiebeeinheit umgewandelt werden; dies geschieht bevorzugt in einer Recheneinheit, die Teil der Steuereinheit sein kann. Die erzeugten Steuerbefehle können der Verschiebeeinheit bereitgestellt werden. Bei der Steuereinheit kann es sich um einen Computer handeln, der einen Prozessor und einen Speicher aufweist.

Der Bediener wird mit einer derartigen Positioniereinrichtung in die Lage versetzt, auf der Darstellungseinheit die Position des Auges zu erkennen und über die Eingabeeinheit die Verschiebeeinheit manuell zu steuern und somit die optische Öffnung des ophthalmologischen Lasertherapiesystems gegenüber dem Auge auszurichten. Zusätzlich oder alternativ kann eine Positionierung automatisch durchgeführt werden. Die auf der Darstellungseinheit dargestellten Aufnahmedaten können dem Bediener bei der Überwachung des Positioniervorgangs und/oder bei der Verifikation des Ergebnisses der Positionierung dienen.

In einer vorteilhaften Ausgestaltung der Positioniereinrichtung umfassen die erste Aufnahmeeinheit eine erste Aufnahmeoptik und die zweite Aufnahmeeinheit eine zweite Aufnahmeoptik. Dabei weist mindestens eine Aufnahmeoptik objektseitig, das heißt am Auge, eine numerische Apertur kleiner als 0,25 auf, bevorzugt kleiner als 0,1, besonders bevorzugt kleiner als 0,05. Auf diese Weise wird das Auge vorteilhaft bereits vor dem Erreichen seiner Zielposition (beispielsweise vor einem Anliegen der Cornea am Kontaktelement) - beispielsweise schon in einem Abstand von 100mm bis 200mm zum Kontaktelement - scharf abgebildet bzw. hinreichend scharf, so dass der Bediener mit einer Positionierung beginnen kann und/oder dass die Steuereinheit Steuerbefehle für die Verschiebeeinheit erzeugen kann.

Typischerweise weist das ophthalmologische Lasertherapiesystem eine Optik auf. Gemäß einer Ausgestaltung der Positioniereinrichtung für ein solches Therapiesystem umfasst die erste Aufnahmeeinheit eine erste Aufnahmeoptik. Dabei sind ein Teil der Optik des ophthalmologisches Lasertherapiesystems und ein Teil der ersten Aufnahmeoptik identisch.

Besonders bevorzugt ist ein Teil einer Laseroptik des Laserschwenkarms bzw. ein Teil einer Untersuchungsoptik des Untersuchungsschwenkarms identisch mit einem Teil der ersten Aufnahmeoptik. D.h. ein Teil der Optik des Laserschwenkarms zur Führung des Laserlichts der Laserquelle ins Auge wird auch von der Aufnahmeoptik einer ersten Aufnahmeeinheit für die Beobachtung des Auges genutzt (bzw. ein Teil der Optik des Untersuchungsschwenkarms). Die gemeinsam genutzte Optik kann die optische Öffnung umfassen. Ein Strahlengang der ersten Aufnahmeeinheit kann unter einem Winkel von weniger als 20° zu einem Strahlengang im Laserschwenkarm bzw. im Untersuchungsschwenkarm ausgebildet sein. Die Strahlengänge können auch koaxial ausgebildet sein.

Um eine kompakte Bauform zu erzielen, weist die erste Aufnahmeoptik vorteilhaft Sensor-seitig geringe Durchmesser und eine hohe F-Number auf.

Der Abbildungsmaßstab der ersten Aufnahmeoptik wird vorzugsweise so gewählt, dass ein gesamtes Behandlungsgebiet oder Untersuchungsgebiet des Auges (z.B. ein Durchmesser von ca. 10mm im korrekt positionierten bzw. angedockten Zustand) scharf abgebildet wird. Dazu kann die Aufnahmeoptik so ausgelegt sein, dass ihr Fokus mit dem Fokus der Laseroptik bzw. Untersuchungsoptik zusammenfällt, z.B. auf oder kurz hinter (also im Auge) einer Abschlussfläche des Kontaktelementes.

Eine Aufnahmeeinheit mit gemeinsam genutzter Optik erlaubt eine "Durchsicht" auf das Auge. Eine Durchsicht ist besonders geeignet, eine Zentrierung des Auges gegenüber der optischen Öffnung zu ermöglichen. Gleichzeitig erlaubt sie eine Beobachtung des Fortschritts der Behandlung während der OP.

Typischerweise wird bei einer gemeinsam genutzten Optik der Strahlengang der Aufnahmeeinheit vom Strahlengang der Laseroptik bzw. Untersuchungsoptik über einen Strahlteiler getrennt (bzw. zusammengeführt). Der Strahlteiler kann als Strahlteilerwürfel ausgeformt sein. Weist die erste Aufnahmeoptik zusätzlich objektseitig eine geringe numerische Apertur (wie oben beschrieben) auf, so kann der Strahlteiler vorteilhaft als schräg im Strahlengang orientierte Planplatte ausgeformt sein. Dabei ist wiederum vorteilhaft der Strahlengang der Aufnahmeeinheit in Transmission geführt und der Strahlengang der Laseroptik bzw. Untersuchungsoptik in Reflexion. Durch die geringe numerische Apertur auf der Seite des Auges bleibt die hohe Qualität der Abbildung der Aufnahmeeinheit erhalten. Die Verwendung einer Planplatte spart Kosten und vor allem Gewicht.

In einer besonders vorteilhaften Weiterentwicklung der beschriebenen Ausgestaltung ist die erste Aufnahmeoptik derart ausgebildet, dass ein Bildfeld eines beobachteten Gebietes mit einem zunehmenden Abstand von der ersten Aufnahmeoptik mit konstanter Bildfeldgröße oder größerer Bildfeldgröße abgebildet wird.

Eine konstante Bildfeldgröße (häufig auch als Objektfeldgröße bezeichnet) kann dadurch erzielt werden, dass die Aufnahmeoptik objektseitig telezentrisch ausgelegt ist. Das bedeutet, dass beispielsweise auch für einem Abstand von 100mm bis 200mm von der optischen Öffnung zum Kontaktelement ein beobachtetes Gebiet mit einer Größe von 10mm Durchmesser abgebildet wird.

Eine größere Bildfeldgröße des beobachteten Gebietes kann durch eine Abweichung von der objektseitigen Telezentrie der Aufnahmeoptik erzielt werden, so dass sich das beobachtete Gebiet bei zunehmendem Abstand vergrößert und ein größerer Teil des Auges oder Patientenkopfes sichtbar wird. Eine Vergrößerung ist vorteilhaft, da sie einen größeren "Fangbereich" ermöglicht; d.h. das Auge befindet sich auch bei größeren Abweichungen von seiner Zielposition gegenüber der optischen Öffnung bereits im Bildfeld der Aufnahmeeinheit.

Die beschriebenen Ausführungen zur ersten Aufnahmeeinheit, die eine Durchsicht bereitstellt, sind auch dazu geeignet, das Auge während einer Lasertherapie zu beobachten und somit die Therapie zu überwachen. Ihr Einsatz ist somit nicht auf den Vorgang einer Positionierung beschränkt.

Auch eine Aufnahmeeinheit, die keine "Durchsicht" bereitstellt, kann eine Aufnahmeoptik aufweisen, die derart ausgebildet ist, dass ein Bildfeld eines beobachteten Gebietes mit einem zunehmenden Abstand von der ersten Aufnahmeoptik mit konstanter Bildfeldgröße oder größerer Bildfeldgröße abgebildet wird. Auch dies ermöglicht eine Verbesserung des "Fangbereichs".

Gemäß einer Ausgestaltung der Positioniereinrichtung weist eine Aufnahmeeinheit in einer Fokusebene ein Bildfeldgröße von mindestens 30mm x 30mm auf, bevorzugt mindestens 40mm x 40mm, besonders bevorzugt mindestens 50mm x 50mm; d.h. ein in der Fokusebene befindliches Objekt dieser Größe wird scharf auf den Sensor der Aufnahmeeinheit abgebildet. Die Fokusebene der Aufnahmeeinheit (bzw. von deren Aufnahmeoptik) befindet sich vorzugsweise in (oder nahe) der Zielposition des Auges. Auch wenn das Behandlungsgebiet oder Untersuchungsgebiet lediglich einen Durchmesser von etwa 10mm aufweist, so ermöglicht die erfindungsgemäße höhere Bildfeldgröße ein Erkennen des Auges auch dann, wenn es sich noch nicht in seiner Zielposition befindet, da das Bildfeld auch das Auge umgebende Strukturen des Patientenkopfes umfasst. Auf diese Weise kann vorteilhaft der "Fangbereich" - über die oben beschriebene Lösung hinaus - weiter verbessert werden.

In einer Ausgestaltung der Positioniereinrichtung weisen die erste Aufnahmerichtung und die zweite Aufnahmerichtung einen Winkel von 90° ± 30° zueinander auf, bevorzugt 90° ± 10°, besonders bevorzugt 90° ± 5°.

Mit anderen Worten werden vom Auge von zwei Aufnahmeeinheiten Aufnahmedaten aus zwei Richtungen im Raum bereitgestellt, die einen Winkel von etwa 90° zueinander aufweisen. Durch diesen Winkel ist es möglich, die Position des Auges genauer zu bestimmen, als es mit einem kleinen Winkel möglich wäre.

Bevorzugt entspricht mindestens eine Aufnahmerichtung einer Bewegungsrichtung der Verschiebeeinheit. Weiterhin ist bevorzugt eine Aufnahmerichtung weitgehend parallel zur Richtung, mit der Laserlicht von der der Optik des Laserschwenkarms zum Auge geführt wird. Besonders bevorzugt weist die Aufnahmeeinheit mit diese Aufnahmerichtung eine Aufnahmeoptik auf, wobei ein Teil der Optik des ophthalmologisches Lasertherapiesystems und ein Teil der ersten Aufnahmeoptik identisch sind.

Bei der ersten Aufnahmerichtung kann es sich beispielsweise um eine Durchsicht handeln, die ein Zentrieren des Auges zur optischen Öffnung besonders gut ermöglicht. Bei der zweiten Aufnahmerichtung kann es sich um eine Seitenansicht auf das Auge handeln. Diese ermöglicht besonders gut eine Annäherung zwischen Auge und optischer Öffnung. Vorteilhaft weist die Seitenansicht in der Fokusebene eine hohe Bildfeldgröße (wie oben beschrieben) auf, um so auch die Teile des Patientenkopfes, die das Auge umgeben (z.B. Augenhöhle, Stirn und Nase), erfassen zu können. Während die Durchsicht eine präzise Zentrierung ermöglicht, erlaubt die Seitenansicht eine genaue (axiale) Annäherung der optischen Öffnung ans Auge (insbesondere, wenn ein großer Fangbereich realisiert ist).

Gemäß einer weiteren Ausgestaltung weist die Positioniereinrichtung eine dritte Aufnahmeeinheit auf, die dazu ausgebildet ist, dritte Aufnahmedaten aus einer dritten Aufnahmerichtung bereitzustellen, wobei die dritte Aufnahmerichtung von der ersten und zweiten Aufnahmerichtung verschieden ist.

Die drei Aufnahmeeinheiten (bzw. deren Aufnahmewinkel gegenüber der Zielposition des Auges) können dazu ein rechtwinkliges Koordinatensystem bilden. Sie können aber auch kleinere (oder größere) Winkel zueinander aufweisen. So reicht es zum Beispiel aus, wenn die dritte Aufnahmeeinheit außerhalb der Ebene angeordnet ist, die von den beiden anderen Aufnahmeeinheiten und der Zielposition des Patientenauges aufgespannt wird, um das Errechnen einer Position des Auges gegenüber der optischen Öffnung in der Steuereinheit (bzw. einer darin ggf. enthaltenen Recheneinheit) zu verbessern. Die dritten Aufnahmedaten können von der ggf. vorhandenen Darstellungseinheit ebenfalls dargestellt werden.

Vorzugsweise weist auch die dritte Aufnahmeeinheit in einer Fokusebene eine hohe Bildfeldgröße auf. Sind zwei Aufnahmeeinheiten mit hohen Bildfeldgrößen ausgestaltet, so wird der "Fangbereich" für eine Positionierung weiter verbessert.

In einer bevorzugten Variante der Positioniereinrichtung weisen die dritte Aufnahmerichtung und die erste Aufnahmerichtung einen Winkel von mehr als 10° und weniger als 90° zueinander aufweisen, bevorzugt zwischen 20° und 70°, besonders bevorzugt zwischen 30° und 60°.

Über die erfindungsgemäßen Winkel wird zum einen sichergestellt, dass die Aufnahmeeinheit das Auge "sehen" kann: Da sich das Auge des Patienten in der Augenhöhle befindet, kann es nicht aus beliebigen Richtungen gesehen werden. Neben einer Durchsicht (z.B. realisiert über die erste Aufnahmeeinheit) und einer Seitenansicht (z.B. über die zweite Aufnahmeeinheit) erlaubt eine zu den beiden Aufnahmerichtungen senkrechte dritte Aufnahmerichtung keine freie Sicht auf das Auge, so dass der genannte Winkel weniger als 90° betragen sollte. Zum anderen wird über die erfindungsgemäßen Winkel eine Verbesserung der Berechnung der relativen Lage zwischen Auge und optischer Öffnung erreicht, da die dritte Aufnahmerichtung eine neue, unabhängige "Blickrichtung" auf das Auge gegenüber der ersten Aufnahmerichtung bietet.

Da eine direkte Sicht des Bedieners auf das Auges des Patienten von Teilen des ophthalmologischen Lasertherapiesystems verdeckt sein kann, wenn die optische Öffnung und das Auge für eine Therapie (annähernd) gut zueinander positioniert sind, ist die dritte Aufnahmeeinheit bevorzugt so am Lasertherapiesystems positioniert, dass die Aufnahmedaten unter einem Beobachtungswinkel (d.h. mit einer Aufnahmerichtung) aufgenommen werden, die der direkten, unverstellten Sicht des Bedieners auf das Auge entspricht. Dies wird über die genannten Winkelbereiche sichergestellt. Die dritte Aufnahmeeinheit ermöglicht somit eine sogenannte "Draufsicht" auf das Auge. Bevorzugt werden diese Aufnahmedaten auch auf der ggf. vorhandenen Darstellungseinheit dargestellt.

Bevorzugt weisen die dritte Aufnahmerichtung und die zweite Aufnahmerichtung einen Winkel von 90° ± 30° zueinander auf, bevorzugt 90° ± 10°, besonders bevorzugt 90° ± 5°.

In einer weiteren Ausgestaltung weist die Positioniereinrichtung eine vierte Aufnahmeeinheit auf, die dazu ausgebildet ist, vierte Aufnahmedaten aus einer vierten Aufnahmerichtung bereitzustellen. Dabei ist die vierte Aufnahmerichtung von der ersten, zweiten und dritten Aufnahmerichtung verschieden. Insbesondere weist die vierte Aufnahmerichtung zur ersten, zweiten oder dritten Aufnahmerichtung einen Winkel von 180° ± 30° auf, bevorzugt 180° ± 10°, besonders bevorzugt 180° ± 5°.

Wenn beispielsweise die zweite Aufnahmeeinheit eine Seitenansicht eines ersten (z.B. rechten) Auges ermöglicht, so kann die vierte Aufnahmeeinheit beispielsweise die Seitenansicht des zweiten (z.B. linken) Auges ermöglichen, ohne dass beispielsweise die Nase des Patienten oder andere Partien des Kopfes eine seitliche Sicht auf das Auge beschränken.

Werden Aufnahmedaten von zwei oder mehr Aufnahmeeinheiten dargestellt, so ist es vorteilhaft, wenn nicht die gesamten Aufnahmedaten angezeigt werden, sondern lediglich jeweils ein Bildausschnitt (eine sogenannte "region of interest", ROI) der Aufnahmedaten. Die ROIs können - beispielsweise von einer Recheneinheit - so gewählt werden, dass die dargestellten Aufnahmedaten das Auge beinhalten. Die ROI der ersten Aufnahmeeinheit, die eine Durchsicht bereitstellt, hat besonders vorteilhaft eine rechteckige, quadratische (mit beispielsweise 900x900 Pixeln) Form oder Kreisform, und die ROIs der zweiten bzw. dritten Aufnahmeeinheit, die eine Seitenansicht bzw. eine Draufsicht anbieten, eine rechteckige Form (mit beispielsweise 700 x 450 Pixeln). Diese ROIs können in Echtzeit (bevorzugt mit einer Latenz zwischen Bildaufnahme und Darstellung von weniger als 0,5 Sekunden) zu einem gängigen Video-Format umgewandelt (z.B. in der Recheneinheit) und von der Darstellungseinheit angezeigt werden. Das resultierende Video hat dann beispielsweise eine Mindestgröße von 1600 x 900 Pixeln.

In einer weiteren Ausgestaltung der Positioniereinrichtung weist die Steuereinheit eine Recheneinheit auf, die dazu ausgebildet ist, Aufnahmedaten einer Aufnahmeeinheit mit einer Zielmarke zu Darstellungsdaten zu verrechnen. Die Steuereinheit kann die Darstellungsdaten bereitstellen.

Bei der Zielmarke kann es sich um eine Markierung handeln, die die Zielposition des Auges gegenüber der optischen Öffnung (z.B. dem Kontaktglas) anzeigt. Diese Markierung kann als Strich, Kreuz, Kreis oder als ein anderes Symbol ausgeformt sein. Bei der Markierung kann es ich auch um eine (stilisierte) Darstellung des Kontaktglases (ggf. samt angrenzender Optik) handeln. Diese Zielmarke kann in der Recheneinheit den Aufnahmedaten überlagert werden und gemeinsam in Darstellungsdaten überführt werden, die auf der Darstellungseinheit angezeigt werden. Die Zielmarke ist bevorzugt so dargestellt, dass sie einen guten Kontrast (z.B. in Helligkeit und/oder Farbe) gegenüber den dargestellten Aufnahmedaten aufweist.

Für eine Überlagerung der Zielmarke mit den Aufnahmedaten kann der Bildinhalt der Aufnahmedaten von der Recheneinheit ausgewertet werden, um in den Daten beispielsweise das Auge, Teile des Auges wie die Iris oder die Pupille und/oder Teile des ophthalmologischen Lasertherapiesystems wie die optische Öffnung (oder das Kontaktglas) zu erkennen.

Die Darstellung der Zielmarke auf der Darstellungseinheit kann die Positionierung des Auges gegenüber der optischen Öffnung verbessern, da dem Bediener visuell Unterstützung für eine manuelle Steuerung der Verschiebeeinheit gegeben wird.

Die Steuereinheit kann dazu ausgebildet sein, auf Basis der Aufnahmedaten Verschiebedaten zu errechnen, die beispielsweise eine Verschieberichtung oder einen Verschiebeweg darstellen. Diese Verschiebedaten können auch auf der Darstellungseinheit dargestellt werden, beispielsweise als Pfeile, die die Verschieberichtung oder den Verschiebeweg markieren - bevorzugt in einer Überlagerung mit Aufnahmedaten. Auf diese Weise kann dem Benutzer angezeigt werden, wie er die Verschiebeeinheit (über die Eingabeeinheit) steuern soll.

In einer weiteren Ausgestaltung weist die Positioniereinrichtung eine Beleuchtungseinheit auf, die dazu ausgebildet ist, das Auge mit Beleuchtungslicht zu beaufschlagen.

Mittels der Beleuchtungseinheit kann die Qualität der Aufnahmedaten verbessert werden, so dass vom Bediener oder der Steuereinheit Steuerbefehle für die Verschiebeeinheit mit höherer Präzision erzeugt werden können.

Die Beleuchtungseinheit kann Beleuchtungslicht beispielsweise über die optische Öffnung (z.B. über das Kontaktglas) führen. Dies kann insbesondere die Bildqualität für eine Durchsicht verbessern. Bei der Beleuchtungseinheit kann es sich auch um eine "flächige" Beleuchtung handeln, die nahe der optischen Öffnung (beispielsweise am Laserschwenkarm oder am Untersuchungsschwenkarm) befestigt ist; dies kann die Bildqualität in Draufsicht verbessern. Es kann sich auch um eine Beleuchtung handeln, die nahe einer Aufnahmeeinheit platziert ist, um von dort das Auge (und den umgebenden Teil des Kopfes) mit Beleuchtungslicht zu beaufschlagen. Es kann sich auch um eine Dunkelfeldbeleuchtung handeln. Bei dem Beleuchtungslicht kann es sich beispielsweise um sichtbares Licht (VIS) oder infrarotes Licht (IR) handeln.

Gemäß einer weiteren Ausgestaltung der Positioniereinrichtung weist die Steuereinheit eine Recheneinheit auf, die dazu ausgebildet ist, unter Verwendung der Aufnahmedaten eine der folgenden Berechnungen durchzuführen: Gesichtserkennung des Patienten, Erkennung eines rechten oder linken Auges des Patienten, Erfassung von Bewegungen des Patienten, Lesen eines Barcodes.

Zur Durchführung der genannten Berechnungen umfasst die Recheneinheit ein entsprechendes Programm, das die Aufnahmedaten analysiert und die Ergebnisse bereitstellt.

Mittels einer Gesichtserkennung kann vorteilhaft die Identität des Patienten überprüft werden. Auf diese Weise können Fehler durch falsche Patientendaten vermieden werden.

Eine Erkennung eines rechten oder linken Auges des Patienten stellt sicher, dass eine Positionierung der optischen Öffnung vor demjenigen Auge erfolgt, das therapiert werden soll. Hier ist eine hohe Bildfeldgröße besonders vorteilhaft, da Aufnahmedaten (bevorzugt in "Draufsicht"), die die Nase des Patienten beinhalten, eine Unterscheidung der Augen vereinfachen. Dies dient ebenfalls der Vermeidung von Fehlern.

Wird ein Fehler erkannt, kann eine Warnung ausgegeben werden.

Eine Nutzung der Aufnahmedaten - beispielsweise aus der Seitenansicht und/oder der Draufsicht - für ein Lesen eines Barcodes oder anderer Codes erlaubt beispielsweise das Erkennen einer Seriennummer auf einem Kontaktelement (oder einer dazugehörigen Verpackung, z.B. ein Data-Matrix-Code). Auch dies dient einer Verbesserung der Qualität einer Lasertherapie durch eine Vermeidung von Fehlern. Hierbei sind wiederum die große Tiefenschärfe der Aufnahmeoptik der Aufnahmeeinheit, eine hohe Bildfeldgröße und/oder ein digitaler Zoom, der durch eine hohen Pixelzahl des Sensors erst möglich wird, von Vorteil.

Neben den Berechnungen, die vorzugsweise vor dem Positionieren des Auges durchgeführt werden können, können auch Patientenbewegungen während der Lasertherapie überwacht werden. Dadurch können gegebenenfalls ein Warnsignal, ein Abbruchsignal oder ein Auslösen eines Abbruchs der Therapie erzeugt werden. Zusätzlich kann das Wohlbefinden des Patienten (vor allem) während der Lasertherapie detektiert werden, die seine Mitarbeit und Kooperation (z.B. ein Blick in ein Fixierlicht) erfordert.

Für die genannten Berechnungen werden bevorzugt Aufnahmedaten einer Draufsicht und/oder einer Seitenansicht verwendet. Die entsprechenden Aufnahmeeinheiten weisen dazu bevorzugt in ihren Fokusebenen eine hohe Bildfeldgröße auf. Da Lösungen nach dem Stand der Technik lediglich Aufnahmedaten in Durchsicht nur auf das Patientenauge selbst, d.h. mit geringer Bildfeldgröße, zur Verfügung stellen, kann die erfindungsgemäße Lösung hier einen besonderen Mehrwert generieren.

Die Positioniereinrichtung kann mehr als eine Darstellungseinheit aufweisen. Beispielsweise kann abseits des Ortes, den der Bediener während der Positionierung und/oder der Therapie einnimmt, eine weitere Darstellungseinheit platziert sein, die einem Assistenten Darstellungsdaten zeigt, so dass auch dieser das Positionieren (und die Therapie) überwachen/beobachten kann.

Die Positioniereinrichtung kann weiterhin eine Darstellungseinheit mit einem Lautsprecher aufweisen. Die Darstellungsdaten können neben den visuell darzustellenden Aufnahmedaten auch akustische Daten (Audiosignale) aufweisen. Bei den akustischen Daten kann es sich um Hinweise oder Warnungen handeln. Die Erzeugung solcher Audiodaten kann in der Recheneinheit erfolgen.

Die Steuereinheit der Positioniereinrichtung weist bevorzugt eine Schnittstelle zu den Aufnahmeeinheiten, der Darstellungseinheit, der Eingabeeinheit, der Verschiebeeinheit und/oder der Beleuchtungseinheit auf. Über diese Schnittstelle (die auch Teilschnittstellen zur Kommunikation mit nur einer oder wenigen Einheiten aufweisen kann) kann ein Datenaustausch zwischen den Einheiten der Positioniereinrichtung ermöglicht werden. Weiterhin kann die Steuereinheit eine Schnittstelle zum ophthalmologischen Lasertherapiesystem aufweisen. Über diese Schnittstelle können Information ausgetauscht werden, wie beispielsweise Patienten- und Behandlungsdaten (oder Warnungen, Zustandsmeldungen). Diese Informationen können - beispielsweise in der Recheneinheit - den Darstellungsdaten hinzugefügt werden.

Gemäß einer bevorzugten Ausgestaltung der Positioniereinrichtung weist die Steuereinheit eine Datenschnittstelle auf. Weiterhin ist die Steuereinheit dazu ausgebildet, einen Datenstrom zu erstellen und über die Datenschnittstelle bereitzustellen. Dabei umfasst der Datenstrom die Aufnahmedaten und/oder die Darstellungsdaten. Die Schnittstelle kann mit einer Speichereinheit verbunden sein, die Teil der Positioniereinrichtung sein kann. Zusätzlich oder alternativ kann die Schnittstelle mit dem ophthalmologischen Lasertherapiesystem verbunden sein, so dass dort der Datenstrom weiterverarbeitet oder in einer Speichereinheit gespeichert werden kann.

Vorteilhaft umfasst der Datenstrom weitere Daten wie Steuerbefehle, Eingabedaten, Informationen der Beleuchtungseinheit oder des ophthalmologischen Lasertherapiesystem (wie beispielsweise Parameter eines Therapielasers oder Patientendaten), Verschiebedaten und/oder weitere Berechnungen aus den Aufnahmedaten. Weisen das Lasertherapiesystem oder die Positioniereinrichtung ein Mikrofon zur Aufnahme von Mikrofondaten (beispielsweise von Gesprächen vor oder während der Therapie) auf, so können diese ebenfalls Teil des Datenstroms sein.

Mit dem Datenstrom (und bevorzugt mit dessen Speichern) wird der Bediener in die Lage versetzt, die Therapie zu dokumentieren. Der (gespeicherte) Datenstrom kann so als Protokoll des Eingriffs dienen. Dabei umfasst der Datenstrom vorzugsweise nicht nur die Vorbereitungen des therapeutischen Eingriffs (wie das Positionieren des Auges bzw. das Andocken an das Kontaktglas), sondern die gesamte Durchführung. Dazu können die Daten im Datenstrom Zeitstempel aufweisen.

Ein zweiter Aspekt der Erfindung betrifft ein ophthalmologisches Lasertherapiesystem, das eine optische Öffnung aufweist. Erfindungsgemäß weist das Lasertherapiesystem eine Positioniereinrichtung nach einer der oben beschriebenen Ausführungen auf.

Ein dritter Aspekt betrifft ein Verfahren für eine Positioniereinrichtung eines ophthalmologischen Lasertherapiesystems, das eine optische Öffnung aufweist, zur Positionierung eines Auges eines Patienten gegenüber der optischen Öffnung. Die Positioniereinrichtung weist eine erste Aufnahmeeinheit auf, die dazu ausgebildet ist, erste Aufnahmedaten vom Auge aus einer ersten Aufnahmerichtung bereitzustellen, sowie eine zweite Aufnahmeeinheit, die dazu ausgebildet ist, zweite Aufnahmedaten vom Auge aus einer zweiten Aufnahmerichtung bereitzustellen. Dabei ist die zweite Aufnahmerichtung von der ersten Aufnahmerichtung verschieden. Weiterhin umfasst die Positioniereinrichtung eine dritte Aufnahmeeinheit, die dazu ausgebildet ist, dritte Aufnahmedaten aus einer dritten Aufnahmerichtung bereitzustellen, wobei die dritte Aufnahmerichtung von der ersten und zweiten Aufnahmerichtung verschieden ist.

Weiterhin umfasst die Positioniereinrichtung eine Verschiebeeinheit, die dazu ausgebildet ist, die relative Lage des Auges gegenüber der optischen Öffnung auf Basis von Steuerbefehlen zu verschieben, und eine Steuereinheit. Das erfindungsgemäße Verfahren weist folgende Schritte auf:
- Empfang von ersten, zweiten und dritten Aufnahmedaten,
- Verrechnen der Aufnahmedaten in Steuerbefehle, und
- Abführen der Steuerbefehle an die Verschiebeeinheit.

Ein alternatives Verfahren für eine Positioniereinrichtung eines ophthalmologischen Lasertherapiesystems, das eine optische Öffnung aufweist, dient der Positionierung eines Auges eines Patienten gegenüber der optischen Öffnung. Die Positioniereinrichtung weist eine erste Aufnahmeeinheit auf, die dazu ausgebildet ist, erste Aufnahmedaten vom Auge aus einer ersten Aufnahmerichtung bereitzustellen, sowie eine zweite Aufnahmeeinheit, die dazu ausgebildet ist, zweite Aufnahmedaten vom Auge aus einer zweiten Aufnahmerichtung bereitzustellen. Dabei ist die zweite Aufnahmerichtung von der ersten Aufnahmerichtung verschieden. Die Positioniereinrichtung umfasst weiterhin eine dritte Aufnahmeeinheit, die dazu ausgebildet ist, dritte Aufnahmedaten aus einer dritten Aufnahmerichtung bereitzustellen, wobei die dritte Aufnahmerichtung von der ersten und zweiten Aufnahmerichtung verschieden ist. Die Positioniereinrichtung umfasst weiterhin eine Darstellungseinheit zur Darstellung der ersten und zweiten Aufnahmedaten vom Auge, und eine Verschiebeeinheit, die dazu ausgebildet ist, die relative Lage des Auges gegenüber der optischen Öffnung auf Basis von Steuerbefehlen zu verschieben. Darüber hinaus weist die Positioniereinrichtung eine Eingabeeinheit, die dazu ausgebildet ist, eine Eingabe von Eingabedaten zu ermöglichen, und eine Steuereinheit auf. Das erfindungsgemäße Verfahren umfasst folgende Schritte:
- Empfang von ersten, zweiten und dritten Aufnahmedaten,
- Darstellung der ersten, zweiten und dritten Aufnahmedaten auf der Darstellungseinheit
- Empfang von Eingabedaten,
- Verrechnen der Eingabedaten in Steuerbefehle, und
- Abführen der Steuerbefehle an die Verschiebeeinheit.

Es sei angemerkt, dass die beschriebenen Verfahren vor der eigentlichen Therapie des Auges vorgenommen werden; sie dienen lediglich der Vorbereitung der Therapie und sind somit nicht Teil davon.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird durch die beigefügten Ansprüche definiert.

Nachfolgend wird die Erfindung beispielsweise anhand der beigefügten Zeichnungen, die auch erfindungswesentliche Merkmale offenbaren, näher erläutert. Es zeigen:
Fig. 1 eine schematische Darstellung eines beispielhaften ophthalmologischen Lasertherapiesystems mit einer Positioniereinrichtung;
Fig. 2 eine schematische Darstellung der Anzeige von Aufnahmedaten auf einer Darstellungseinheit;
Fig. 3a, 3b, 3c Schnittbilder eines Ausführungsbeispiels der Aufnahmeoptik einer Aufnahmeeinheit;
Fig. 4 eine schematische Darstellung eines Ausführungsbeispiels einer Positioniereinrichtung.

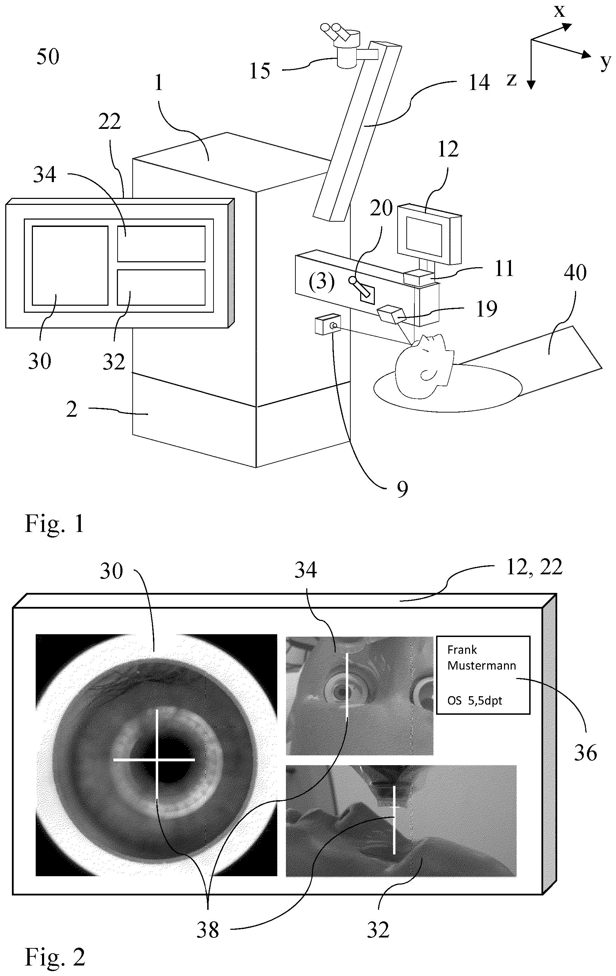

In der Fig. 1 ist eine schematische Darstellung eines beispielhaften ophthalmologischen Lasertherapiesystems 50 gezeigt.

Das Beispiel des ophthalmologischen Lasertherapiesystems 50 setzt sich zusammen aus einer Gerätebasis 2 und einem auf dieser Gerätebasis 2 in der Höhe über einer Bodenebene, also der z-Richtung, und in seiner Position in der Ebene, also in x- und y-Richtung, verstellbaren Gerätekopf 1. Der Gerätekopf 1 enthält einen ersten Teil der zur Durchführung der Lasertherapie erforderlichen Laseroptik. Der Gerätekopf 1 enthält im gezeigten Beispiel auch die zur Erzeugung eines entsprechenden gepulsten Laserstrahls erforderliche Laserquelle, die hier eine Femtosekunden-Laserquelle ist.

Der zweite Teil der Laseroptik befindet sich in einem Laserschwenkarm 3. Dieser kann um eine horizontale Achse (nicht eingezeichnet) von einer Ruhestellung, in der er ungefähr senkrecht nach oben ragt, in eine Arbeitsstellung, in der er etwa waagerecht am Gerätekopf 1, also etwa parallel zur Bodenebene, angeordnet ist, sowie zurück geschwenkt werden. Der Laserschwenkarm 3 kann also für die Lasertherapie am Patientenauge über den Patienten geschwenkt werden. In Schritten jedoch, in denen der Laserschwenkarm 3 nicht benötigt wird, kann er in eine Ruhestellung zurückverbracht werden, um den Raum über der Arbeitsstellung anderweitig zu nutzen. Am Laserschwenkarm 3 befindet sich eine optische Öffnung, nämlich die Laseraustrittsöffnung, von der aus ein Therapielaserstrahl zum Therapieort im Auge eines Patienten geleitet wird (wenn sich der Laserschwenkarm 3 - wie in der Fig. 1 dargestellt - in der Arbeitsstellung befindet). Die Laseraustrittsöffnung ist innerhalb des Laserschwenkarms 3 beweglich angeordnet.

Eine erste Aufnahmeeinheit der Positioniereinrichtung ist als Kamera 11 ausgebildet, die sich am Laserschwenkarm 3 befindet. Sie weist einen optischen Strahlengang zum Patientenauge auf, der abschnittsweise mit dem Strahlengang für die Therapiestrahlung identisch ist. D.h. ein Teil der Laseroptik im Laserschwenkarm 3 ist identisch mit einem Teil der Optik der Aufnahmeoptik von Kamera 11. Das Kontaktglas (nicht eingezeichnet) ist Teil dieser gemeinsam genutzten Optik. Die Kamera 11 erlaubt die Aufnahme von Aufnahmedaten des Patientenauges in einer "Durchsicht". Die Positioniereinrichtung weist weitere Kameras 9, 19 (zweite und dritte Aufnahmeeinheit) auf. Diese sind dazu ausgebildet, Aufnahmedaten vom Patienten, d.h. vom Patientenauge sowie von dem Auge umgebenden Kopfteilen, unter Aufnahmerichtungen zu erzeugen, die von der Aufnahmerichtung von Kamera 11 abweichen. Zusätzlich sind auch die Aufnahmerichtungen von Kamera 9 und 19 unterschiedlich. Kamera 9 erlaubt eine "Seitenansicht" des Patientenauges; die Aufnahmerichtungen zwischen Kamera 11 und 9 weisen zueinander einen Winkel von etwa 90° auf (gegenüber der Zielposition des Auges). Kamera 19 erlaubt eine "Draufsicht" auf den Patienten und das Patientenauge. Der Winkel zwischen den Aufnahmerichtungen der zweiten Aufnahmeeinheit (Kamera 9) und der dritten Aufnahmeeinheit (Kamera 19) beträgt ebenfalls etwa 90°. Der Winkel zwischen den Aufnahmerichtungen der ersten Aufnahmeeinheit (Kamera 11) und der dritten Aufnahmeeinheit (Kamera 19) beträgt etwa 55°. Die Kameras 11, 9 und 19 spannen ein Koordinatensystem auf, mit dem der Kopf und das Patientenauge dreidimensional erfasst werden können. Aufgrund der Anordnungen von Kamera 11 und Kamera 9 kann die dreidimensionale Position des Auges bereits über diese beiden Kameras erfasst werden; die Bestimmung der Position des Auges wird bei Verwendung der Aufnahmedaten von Kamera 19 genauer bzw. für einen Benutzer, der eine manuelle Positionierung vornehmen möchte, intuitiver.

Die Verschiebeeinheit (hier Teil der Patientenliege 40) ist dazu ausgebildet, eine Verschiebung des Gerätekopfes 1 gegenüber der Gerätebasis 2 zu kontrollieren. Weiterhin erlaubt sie eine Verschiebung der Laseraustrittsöffnung am Laserschwenkarm 3. Auf diese Weise lässt sich die Laseraustrittsöffnung gegenüber dem Patientenauge in allen drei Raumdimensionen verschieben.

Die Eingabeeinheit 20 ist als Joystick ausgebildet. Er befindet sich am Laserschwenkarm 3. Über den Joystick kann der Bediener Eingabedaten erzeugen (durch Bewegung des Joysticks oder Auslösen daran befindlicher Schalter oder Regler). Die Eingabedaten werden an eine Steuereinheit (nicht dargestellt) abgeführt und von dieser (in einer Recheneinheit, nicht dargestellt) in Steuerbefehle umgesetzt. Die Steuerbefehle wiederum werden an die Verschiebeeinheit abgeführt. Die Steuereinheit (mit Recheneinheit) befindet sich in diesem Beispiel im Gerätekopf 1.

Die Darstellungseinheit der Positioniereinrichtung ist als Monitor 12 ausgebildet und ist ebenfalls am Laserschwenkarm 3 befestigt. Sie ist über eine Drehachse (nicht eingezeichnet) mit dem Laserschwenkarm 3 verbunden, um eine horizontale Ausrichtung des Monitors 12 sowohl für die Ruhestellung als auch die Arbeitsstellung zu gewährleisten. Der Monitor 12 ist als Touch-Screen ausgebildet; die Eingabeeinheit 20 könnte somit auch in der Darstellungseinheit integriert sein.

Die Positioniereinrichtung umfasst zusätzlich eine weitere Darstellungseinheit 22, die seitlich am Gerätekopf 1 befestigt ist. Während sich Monitor 12 in einer für den Chirurgen (Bediener) ergonomischen Position befindet, ist Monitor 22 so platziert, dass er weiteren Beobachtern der Therapie eine gute Sicht ermöglicht.

Die Darstellungseinheiten 12, 22 zeigen die Aufnahmedaten der Kameras 11, 9 und 19 für die Durchsicht 30, die Seitenansicht 32 und die Draufsicht 34 auf das Pateientenauge an.

Das Lasertherapiesystem 50 weist weiterhin einen selbständigen Untersuchungsschwenkarm 14 auf, der über eine Schwenkachse (nicht eingezeichnet) bewegt werden kann. Der Untersuchungsschwenkarmen 14 kann ebenfalls zwischen einer Ruhestellung und einer Arbeitsstellung hin- und hergeschwenkt werden. Mit dem Untersuchungsschwenkarm 14 ist über eine drehbare Achse (nicht eingezeichnet) ein Operationsmikroskop 15 verbunden. Die Schwenkachse und die drehbare Achse sind so ausgestaltet, dass der Arbeitsort des Operationsmikroskop 15 in der Arbeitsstellung mit dem Therapieort zusammenfällt. Alternativ oder zusätzlich zu einer Platzierung der ersten Aufnahmeeinheit 11 am Laserschwenkarm 3 ist es möglich, eine Aufnahmeeinheit am Untersuchungsschwenkarmen 14 zu befestigen. Sie kann dabei beispielsweise so ausgebildet sein, dass sie einen optischen Strahlengang zum Patientenauge aufweist, der abschnittsweise mit dem Strahlengang für Untersuchungsstrahlung identisch ist. D.h. ein Teil der Untersuchungsoptik im Untersuchungsschwenkarmen 14 ist identisch mit einem Teil der Optik der Aufnahmeoptik der Kamera. Die Aufnahmedaten dieser Kamera können ebenfalls nach dem Wechsel vom Laserschwenkarm 3 über dem Patientenauge zum Untersuchungsschwenkarm 14 über dem Patientenauge (Untersuchungsschwenkarm 14 befindet sich in Arbeitsstellung, Laserschwenkarm 3 befindet sich in Ruhestellung) auf der Darstellungseinheit 12, 22 dargestellt werden und/oder in einer Speichereinheit zur Dokumentation abgespeichert werden. Die gespeicherten Daten der Dokumentation können auch Audiosignale, Mikrofondaten oder Patienten- und Behandlungsdaten aufweisen. Die auf den Darstellungseinheiten 12, 22 dargestellten Aufnahmedaten können danach ausgewählt sein, welcher Schwenkarm sich in seiner Arbeitsstellung befindet. Vorzugsweise werden nur die Aufnahmedaten derjenigen Aufnahmeeinheit in Durchsicht gezeigt, die mit dem jeweiligen Schwenkarm verknüpft sind, der sich gerade in seiner Arbeitsstellung befindet.

Es sei angemerkt, dass die erfindungsgemäße Positioniereinrichtung nicht auf eine Verwendung in einem ophthalmologischen Lasertherapiesystem 50 mit einem Laserschwenkarm 3 und einem Untersuchungsschwenkarm 14 beschränkt ist. Vielmehr ist eine Nutzung in einem Lasertherapiesystem 50 ebenfalls möglich und vorteilhaft, das über einen Laserschwenkarm 3 verfügt, der zwar positioniert, aber nicht in eine Ruhestellung verbracht werden kann, und/oder das über keinen Untersuchungsschwenkarm 14 verfügt.

In Fig. 2 ist eine schematische Darstellung der Anzeige von Aufnahmedaten auf einer Darstellungseinheit 12, 22 gezeigt. Die Darstellungseinheiten 12 und 22 werden erfindungsgemäß benutzt, um dem Bediener während der Positionierung des Patientenauges gegenüber der optischen Öffnung (z.B. der Laseraustrittsöffnung und/oder Kontaktglas) sowohl eine Draufsicht 34, gewonnen mit Kamera 19, eine Seitenansicht 32, erzeugt mit Kamera 9, als auch die Durchsicht 30, beobachtet durch das Kontaktglas hindurch auf das Patientenauge, erzeugt mit Kamera 11, anzuzeigen. Kamera 19 und die Anzeige ihres Bildes 34 auf dem Monitor 12 erhöht die Ergonomie für den Chirurgen, da der Patient während der OP teilweise durch den Laserschwenkarm 3, der die Laseroptik enthält, verdeckt wird. Vorteilhaft werden die Aufnahmedaten (beispielsweise der Draufsicht) auch nach dem Positionieren des Auges angezeigt, beispielsweise zur Überwachung der Therapie.

Den dargestellten Aufnahmedaten sind Zielmarken 38 überlagert. Mit Hilfe der Zielmarken 38 kann die Positionierung des Auges gegenüber der optischen Öffnung verbessert werden. Insbesondere ist für den Bediener schnell und intuitiv ersichtlich, in welche Richtung er Joystick 20 bewegen/steuern muss, damit das Patientenauge in seine Zielposition gelangt.

Zusätzlich werden auf der Darstellungseinheit 12, 22 Informationsdaten 36 dargestellt. Diese werden vom Lasertherapiesystem der Steuereinheit über eine Schnittstelle zur Verfügung gestellt. Weiterhin ist eine Ausgabe von Warnungen, die vom Lasertherapiesystem oder der Steuereinheit (bzw. der Recheneinheit) bereitgestellt werden, möglich.

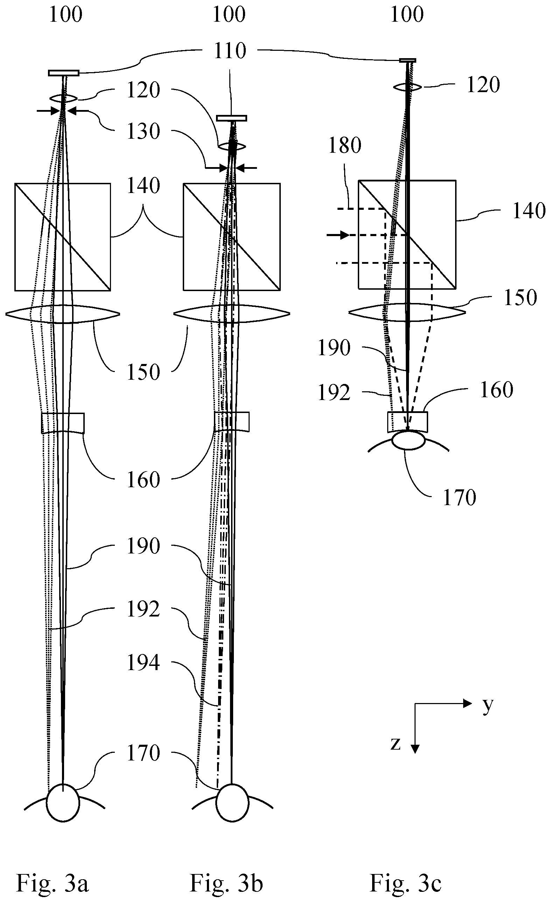

In Fig. 3a, 3b, 3c sind Schnittbilder der Aufnahmeoptik einer Aufnahmeeinheit 100 dargestellt. Dabei handelt es sich um eine Aufnahmeeinheit 100, die eine Aufnahmeoptik aufweist, die teilweise mit der Laseroptik des ophthalmologischen Lasertherapiesystems identisch ist. Dargestellt sind ein Auge 170, ein Objektiv 150 mit einem Kontaktelement 160 für die Laserbehandlung, ein Strahlteiler 140 und ein Kameraobjektiv 120 mit Blende 130 sowie ein Sensor 110 (CCD- oder CMOS-Detektor). Dabei werden das Kontaktelement 160, das Objektiv 150 und der Strahlteiler 140 von der Laseroptik und der Aufnahmeoptik gemeinsam genutzt. Das optische Design der Aufnahmeoptik zeichnet sich dadurch aus, dass es eine geringe numerische Apertur aufweisen, um so eine hohe Tiefenschärfe zu gewährleisten. Auf diese Weise wird das Auge 170 bereits im Abstand von 100mm bis 200m vom Kontaktelement 160 so scharf abgebildet, dass eine Zentrierung des Auges 170 ermöglicht wird. Der Abbildungsmaßstab sowie die freien Durchmesser der optischen Elemente sind so gewählt, dass am Kontaktelement 160, das die Zielposition des Auges ist, ein Bildfeld mit einem Durchmesser von ca. 10mm scharf abgebildet wird.

Der Strahlverlauf vom Sensor 110 bis zum Kontaktelement 160 ist in Fig. 3a so ausgebildet, dass das Gebiet von 10mm Durchmesser auch für Abstände von 100mm bis 250mm gleich groß abgebildet wird. Dazu ist die Blende 130 so positioniert, dass eine objektseitige Telezentrie gewährleistet ist. Dies wird durch das Strahlbündel 190 (durchgezogene Linien) für das Zentrum des abzubildenden Gebietes und durch das Strahlbündel 192 (gepunktete Linie) für einen Randbereich des abzubildenden Gebietes verdeutlicht. In Fig. 3a verlaufen die Strahlenbündel 190 und 192 parallel, und ändern ihren Abstand nicht.

In Fig. 3b ist eine vorteilhafte Variante dazu dargestellt. Hier ist das optische Design so ausgelegt, dass sich das Bildfeld des abgebildeten Gebietes bei einem größeren Abstand vom Kontaktelement 160 vergrößert, so dass ein größerer Teil des Auges bzw. des Patientenkopfes auf den Kamerasensor 110 abgebildet wird. Dies erlaubt ein verbessertes "Einfangen" des Auges zu Beginn einer Positionierung, wenn sich Auge 170 und Kontaktelement 160 noch in großem Abstand voneinander befinden. Dies wird durch das Strahlbündel 190 (durchgezogene Linien) für das Zentrum des abzubildenden Gebietes, durch das Strahlbündel 192 (gepunktete Linie) für einen Randbereich sowie durch das Strahlbündel 194 (Linie aus Punkten und Strichen) für einen mittleren Bereich des abzubildenden Gebietes verdeutlicht. Der Abstand zwischen den Strahlenbündeln 190, 192 und 194 wächst mit dem Abstand zum Kontaktelement 160.

Das Kontaktelement 160 kann als planes oder gekrümmtes (wie hier gezeigt) Kontaktglas ausgeformt sein. Die Telezentrie (bzw. Abweichung davon) wird über den Abstand zwischen Objektiv 150 und Kameraobjektiv 120 bzw. dessen Blende 130 bestimmt.

Der Strahlengang der Aufnahmeeinheit und der Laseroptik werden im Strahlteiler 140 zusammengeführt. Dieser ist in den Fig. 3a, 3b, 3c als Strahlteilerwürfel ausgebildet. Eine Ausführung als Teilerplatte ist jedoch auch möglich.

In Fig. 3c ist zur Verdeutlichung der Strahlverlauf der Therapiestrahlung 180 für die gemeinsam genutzte Optik als gestrichelte Linie eingezeichnet. Hier befindet sich das Auge 170 in seiner Zielposition; es ist in Kontakt mit dem Kontaktelement 160.

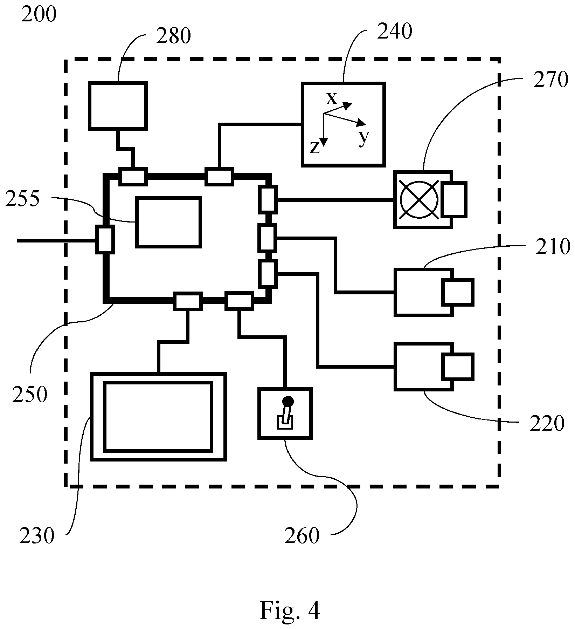

In Fig. 4 ist eine schematische Darstellung eines Ausführungsbeispiels einer Positioniereinrichtung 200 dargestellt. Die Positioniereinrichtung 200 weist eine erste Aufnahmeeinheit 210, eine zweite Aufnahmeeinheit 220, eine Darstellungseinheit 230 und eine Verschiebeeinheit 240 auf. Weiterhin umfasst das Ausführungsbeispiel der Positioniereinrichtung 200 eine Eingabeeinheit 260, eine Beleuchtungseinheit 270 sowie eine Speichereinheit 280. Ein Datenaustausch der Einheiten zur Steuereinheit 250 wird über Schnittstellen (als Kästen an der Steuereinheit dargestellt) realisiert. Der Datenaustausch erfolgt hier über Kabel; er kann auch kabellos erfolgen. Weiterhin ist eine Schnittstelle eingerichtet für einen Austausch von Daten mit dem Lasertherapiesystem 50 (dargestellt über eine Leitung, die die mittels gestrichelter Linie dargestellte Positioniereinrichtung 200 verlässt). Die Steuereinheit 250 weist eine Recheneinheit 255, die die Berechnung von Verschiebedaten erlaubt.

Die vorstehend genannten und in verschiedenen Ausführungsbeispielen beschriebenen Merkmale der Erfindung sind dabei nicht nur in den angegebenen beispielhaften Kombinationen, sondern auch in anderen Kombinationen oder allein einsetzbar, ohne den Rahmen der vorliegenden Erfindung zu verlassen.

Eine auf Verfahrensmerkmale bezogene Beschreibung einer Vorrichtung gilt bezüglich dieser Merkmale analog für das entsprechende Verfahren, während Verfahrensmerkmale entsprechend funktionelle Merkmale der beschriebenen Vorrichtung darstellen.

## Patentansprüche

1. Positioniereinrichtung (200) für ein ophthalmologisches Lasertherapiesystem (50), das eine optische Öffnung für einen Austritt von Licht einer Laserquelle aus dem ophthalmologische Lasertherapiesystem aufweist, zur Positionierung eines Auges (170) eines Patienten gegenüber der optischen Öffnung, wobei die Positioniereinrichtung (200)
- eine erste Aufnahmeeinheit (11, 100, 210), die dazu ausgebildet ist, erste Aufnahmedaten vom Auge (170) aus einer ersten Aufnahmerichtung bereitzustellen,
- eine zweite Aufnahmeeinheit (9, 100, 220), die dazu ausgebildet ist, zweite Aufnahmedaten vom Auge (170) aus einer zweiten Aufnahmerichtung bereitzustellen,
wobei die zweite Aufnahmerichtung von der ersten Aufnahmerichtung verschieden ist,
- eine dritte Aufnahmeeinheit (19), die dazu ausgebildet ist, dritte Aufnahmedaten aus einer dritten Aufnahmerichtung bereitzustellen, wobei die dritte Aufnahmerichtung von der ersten und zweiten Aufnahmerichtung verschieden ist,
- eine Verschiebeeinheit (240), die dazu ausgebildet ist, die relative Lage des Auges (170) gegenüber der optischen Öffnung auf Basis von Steuerbefehlen zu verschieben, und
- eine Steuereinheit, die dazu ausgebildet ist, Steuerbefehle auf Basis der ersten, zweiten und dritten Aufnahmedaten zu erzeugen und der Verschiebeeinheit (240) bereitzustellen,
umfasst.

2. Positioniereinrichtung (200) für ein ophthalmologisches Lasertherapiesystem (50), das eine optische Öffnung für einen Austritt von Licht einer Laserquelle aus dem ophthalmologische Lasertherapiesystem aufweist, zur Positionierung eines Auges (170) eines Patienten gegenüber der optischen Öffnung, wobei die Positioniereinrichtung (200)
- eine erste Aufnahmeeinheit (11, 100, 210), die dazu ausgebildet ist, erste Aufnahmedaten vom Auge (170) aus einer ersten Aufnahmerichtung bereitzustellen,
- eine zweite Aufnahmeeinheit (9, 100, 220), die dazu ausgebildet ist, zweite Aufnahmedaten vom Auge (170) aus einer zweiten Aufnahmerichtung bereitzustellen,
wobei die zweite Aufnahmerichtung von der ersten Aufnahmerichtung verschieden ist,
- eine dritte Aufnahmeeinheit (19), die dazu ausgebildet ist, dritte Aufnahmedaten aus einer dritten Aufnahmerichtung bereitzustellen, wobei die dritte Aufnahmerichtung von der ersten und zweiten Aufnahmerichtung verschieden ist,
- eine Darstellungseinheit (12, 22, 230) zur Darstellung der ersten, zweiten und dritten Aufnahmedaten vom Auge (170),
- eine Verschiebeeinheit (240), die dazu ausgebildet ist, die relative Lage des Auges (170) gegenüber der optischen Öffnung auf Basis von Steuerbefehlen zu verschieben,
- eine Eingabeeinheit (20, 260), die dazu ausgebildet ist, eine Eingabe von Eingabedaten zu ermöglichen, und
- eine Steuereinheit (250), die dazu ausgebildet ist, Steuerbefehle auf Basis der Eingabedaten zu erzeugen, oder Steuerbefehle auf Basis der Eingabedaten und auf Basis der Aufnahmedaten zu erzeugen, und die Steuerbefehle der Verschiebeeinheit (240) bereitzustellen,
aufweist.

3. Positioniereinrichtung (200) nach einem der Ansprüche 1 oder 2, **dadurch gekennzeichnet, dass** die erste Aufnahmeeinheit (11, 100, 210) eine erste Aufnahmeoptik und die zweite Aufnahmeeinheit (9, 100, 220) eine zweite Aufnahmeoptik umfassen, wobei mindestens eine Aufnahmeoptik objektseitig eine numerische Apertur kleiner als 0,25 aufweist, bevorzugt kleiner als 0,1, besonders bevorzugt kleiner als 0,05.

4. Positioniereinrichtung (200) nach einem der Ansprüche 1 bis 3, wobei das ophthalmologische Lasertherapiesystem (50) eine Optik aufweist, **dadurch gekennzeichnet, dass** die erste Aufnahmeeinheit (11, 100, 210) eine erste Aufnahmeoptik umfasst, wobei ein Teil der Optik des ophthalmologisches Lasertherapiesystems (50) und ein Teil der ersten Aufnahmeoptik identisch sind.

5. Positioniereinrichtung (200) nach einem der Ansprüche 3 oder 4, **dadurch gekennzeichnet, dass** die erste Aufnahmeoptik derart ausgebildet ist, dass ein Bildfeld eines beobachteten Gebietes mit einem zunehmenden Abstand von der ersten Aufnahmeoptik mit konstanter Bildfeldgröße oder größerer Bildfeldgröße abgebildet wird.

6. Positioniereinrichtung (200) nach einem der Ansprüche 1 bis 5, **dadurch gekennzeichnet, dass** eine Aufnahmeeinheit in einer Fokusebene ein Bildfeldgröße von mindestens 30mm x 30mm aufweist, bevorzugt mindestens 40mm x 40mm, besonders bevorzugt mindestens 50mm x 50mm.

7. Positioniereinrichtung (200) nach einem der Ansprüche 1 bis 6, **dadurch gekennzeichnet, dass** die erste Aufnahmerichtung und die zweite Aufnahmerichtung einen Winkel von 90° ± 30° zueinander aufweisen, bevorzugt 90° ± 10°, besonders bevorzugt 90° ± 5°.

8. Positioniereinrichtung (200) nach einem der Ansprüche 1 bis 7, **dadurch gekennzeichnet, dass** die dritte Aufnahmerichtung und die erste Aufnahmerichtung einen Winkel zwischen 10° und 90° zueinander aufweisen, bevorzugt zwischen 20° und 70°, besonders bevorzugt zwischen 30° und 60°.

9. Positioniereinrichtung (200) nach einem der Ansprüche 1 bis 8, **dadurch gekennzeichnet, dass** die Steuereinheit (250) eine Recheneinheit (255) aufweist, die dazu ausgebildet ist, Aufnahmedaten einer Aufnahmeeinheit (100, 210, 220) mit einer Zielmarke (38) zu Darstellungsdaten zu verrechnen, und dass die Steuereinheit (250) die Darstellungsdaten bereitstellt.

10. Positioniereinrichtung (200) nach einem der Ansprüche 1 bis 9, **dadurch gekennzeichnet, dass** das Positioniereinrichtung (200) eine Beleuchtungseinheit (270) aufweist, die dazu ausgebildet ist, das Auge (170) mit Beleuchtungslicht zu beaufschlagen.

11. Positioniereinrichtung (200) nach einem der Ansprüche 1 bis 10, **dadurch gekennzeichnet, dass** die Steuereinheit (250) eine Recheneinheit (255) aufweist, die dazu ausgebildet ist, unter Verwendung der Aufnahmedaten eine der folgenden Berechnungen durchzuführen:
- Gesichtserkennung des Patienten,
- Erkennung eines rechten oder linken Auges (170) des Patienten,
- Lesen eines Barcodes,
- Erfassung von Bewegungen des Patienten.

12. Positioniereinrichtung (200) nach einem der Ansprüche 1 bis 11, **dadurch gekennzeichnet, dass** die Steuereinheit (255) eine Datenschnittstelle aufweist und dass die Steuereinheit dazu ausgebildet ist, einen Datenstrom zu erstellen, der die Aufnahmedaten und/oder Darstellungsdaten umfasst, und den Datenstrom über die Datenschnittstelle bereitzustellen.

13. Ophthalmologisches Lasertherapiesystem (50), das eine optische Öffnung für einen Austritt von Licht einer Laserquelle aus dem ophthalmologische Lasertherapiesystem aufweist und das eine Positioniereinrichtung (200) nach einem der Ansprüche 1 bis 12 aufweist.

14. Verfahren für eine Positioniereinrichtung (200) eines ophthalmologischen Lasertherapiesystems (50), das eine optische Öffnung für einen Austritt von Licht einer Laserquelle aus dem ophthalmologische Lasertherapiesystem aufweist, zur Positionierung eines Auges (170) eines Patienten gegenüber der optischen Öffnung, wobei die Positioniereinrichtung (200)
- eine erste Aufnahmeeinheit (11, 100, 210), die dazu ausgebildet ist, erste Aufnahmedaten vom Auge (170) aus einer ersten Aufnahmerichtung bereitzustellen,
- eine zweite Aufnahmeeinheit (9, 100, 220), die dazu ausgebildet ist, zweite Aufnahmedaten vom Auge (170) aus einer zweiten Aufnahmerichtung bereitzustellen,
wobei die zweite Aufnahmerichtung von der ersten Aufnahmerichtung verschieden ist,
- eine dritte Aufnahmeeinheit (19), die dazu ausgebildet ist, dritte Aufnahmedaten aus einer dritten Aufnahmerichtung bereitzustellen, wobei die dritte Aufnahmerichtung von der ersten und zweiten Aufnahmerichtung verschieden ist,
- eine Verschiebeeinheit (240), die dazu ausgebildet ist, die relative Lage des Auges (170) gegenüber der optischen Öffnung auf Basis von Steuerbefehlen zu verschieben, und
- eine Steuereinheit (250)
aufweist, und wobei das Verfahren folgende Schritte umfasst:
- Empfang von ersten, zweiten und dritten Aufnahmedaten,
- Verrechnen der ersten, zweiten und dritten Aufnahmedaten in Steuerbefehle und
- Abführen der Steuerbefehle an die Verschiebeeinheit (240).

15. Verfahren für eine Positioniereinrichtung (200) eines ophthalmologischen Lasertherapiesystems (50), das eine optische Öffnung für einen Austritt von Licht einer Laserquelle aus dem ophthalmologische Lasertherapiesystem aufweist, zur Positionierung eines Auges (170) eines Patienten gegenüber der optischen Öffnung, wobei die Positioniereinrichtung (200)
- eine erste Aufnahmeeinheit (11, 100, 210), die dazu ausgebildet ist, erste Aufnahmedaten vom Auge (170) aus einer ersten Aufnahmerichtung bereitzustellen,
- eine zweite Aufnahmeeinheit (9, 100, 220), die dazu ausgebildet ist, zweite Aufnahmedaten vom Auge (170) aus einer zweiten Aufnahmerichtung bereitzustellen,
wobei die zweite Aufnahmerichtung von der ersten Aufnahmerichtung verschieden ist,
- eine dritte Aufnahmeeinheit (19), die dazu ausgebildet ist, dritte Aufnahmedaten aus einer dritten Aufnahmerichtung bereitzustellen, wobei die dritte Aufnahmerichtung von der ersten und zweiten Aufnahmerichtung verschieden ist,
- eine Darstellungseinheit (12, 22, 230) zur Darstellung der ersten, zweiten und dritten Aufnahmedaten vom Auge (170),
- eine Verschiebeeinheit (240), die dazu ausgebildet ist, die relative Lage des Auges (170) gegenüber der optischen Öffnung auf Basis von Steuerbefehlen zu verschieben,
- eine Eingabeeinheit (20, 260), die dazu ausgebildet ist, eine Eingabe von Eingabedaten zu ermöglichen, und
- eine Steuereinheit (250)
aufweist, und wobei das Verfahren folgende Schritte umfasst:
- Empfang von ersten, zweiten und dritten Aufnahmedaten,
- Darstellung der ersten, zweiten und dritten Aufnahmedaten auf der Darstellungseinheit (12, 22, 230),
- Empfang von Eingabedaten,
- Verrechnen der Eingabedaten in Steuerbefehle und
- Abführen der Steuerbefehle an die Verschiebeeinheit (240).

## Claims

1. Positioning device (200) for an ophthalmological laser therapy system (50) comprising an optical opening allowing light from a laser source to exit the ophthalmological laser therapy system, for positioning a patient's eye (170) vis-à-vis the optical opening, the positioning device (200) comprising
- a first recording unit (11, 100, 210) designed to provide first recording data of the eye (170) from a first recording direction,
- a second recording unit (9, 100, 220) designed to provide second recording data of the eye (170) from a second recording direction,
the second recording direction differing from the first recording direction,
- a third recording unit (19) designed to provide third recording data from a third recording direction, the third recording direction differing from the first and second recording directions,
- a displacement unit (240) designed to displace the relative position of the eye (170) vis-à-vis the optical opening on the basis of control commands, and
- a control unit designed to generate control commands on the basis of the first, second and third recording data and provide these control commands to the displacement unit (240).

2. Positioning device (200) for an ophthalmological laser therapy system (50) comprising an optical opening allowing light from a laser source to exit the ophthalmological laser therapy system, for positioning a patient's eye (170) vis-à-vis the optical opening, the positioning device (200) comprising
- a first recording unit (11, 100, 210) designed to provide first recording data of the eye (170) from a first recording direction,
- a second recording unit (9, 100, 220) designed to provide second recording data of the eye (170) from a second recording direction,
the second recording direction differing from the first recording direction,
- a third recording unit (19) designed to provide third recording data from a third recording direction, the third recording direction differing from the first and second recording directions,
- a display unit (12, 22, 230) for displaying the first, second and third recording data of the eye (170),
- a displacement unit (240) designed to displace the relative position of the eye (170) vis-à-vis the optical opening on the basis of control commands,
- an input unit (20, 260) designed to enable an input of input data, and
- a control unit (250) designed to generate control commands on the basis of the input data, or generate control commands on the basis of the input data and on the basis of the recording data, and provide the control commands to the displacement unit (240).

3. Positioning device (200) according to either of Claims 1 and 2, **characterized in that** the first recording unit (11, 100, 210) comprises a first recording optical unit and the second recording unit (9, 100, 220) comprises a second recording optical unit, at least one recording optical unit having on the object side a numerical aperture of less than 0.25, preferably less than 0.1, particularly preferably less than 0.05.

4. Positioning device (200) according to any of Claims 1 to 3, the ophthalmological laser therapy system (50) comprising an optical unit, **characterized in that** the first recording unit (11, 100, 210) comprises a first recording optical unit, a part of the optical unit of the ophthalmological laser therapy system (50) and a part of the first recording optical unit being identical.

5. Positioning device (200) according to either of Claims 3 and 4, **characterized in that** the first recording optical unit is designed such that an image field of an observed area is imaged with a constant image field size or a larger image field size with increasing distance from the first recording optical unit.

6. Positioning device (200) according to any of Claims 1 to 5, **characterized in that** a recording unit has an image field size of at least 30 mm x 30 mm, preferably at least 40 mm x 40 mm, particularly preferably at least 50 mm x 50 mm in a focal plane.

7. Positioning device (200) according to any of Claims 1 to 6, **characterized in that** the first recording direction and the second recording direction form an angle of 90° ± 30° with each other, preferably 90° ± 10°, particularly preferably 90° ± 5°.

8. Positioning device (200) according to any of Claims 1 to 7, **characterized in that** the third recording direction and the first recording direction form an angle of between 10° and 90° with each other, preferably between 20° and 70°, particularly preferably between 30° and 60°.

9. Positioning device (200) according to any of Claims 1 to 8, **characterized in that** the control unit (250) comprises a computing unit (255) designed to combine recording data from a recording unit (100, 210, 220) with a target mark (38) by calculation to form display data, and **in that** the control unit (250) provides the display data.

10. Positioning device (200) according to any of Claims 1 to 9, **characterized in that** the positioning device (200) comprises an illumination unit (270) designed to impinge the eye (170) with illumination light.

11. Positioning device (200) according to any of Claims 1 to 10, **characterized in that** the control unit (250) comprises a computing unit (255) designed to carry out any one of the following calculations using the recording data:
- facial recognition of the patient,
- recognition of a patient's right or left eye (170),
- reading of a barcode,
- detection of movements of the patient.

12. Positioning device (200) according to any of Claims 1 to 11, **characterized in that** the control unit (255) comprises a data interface and **in that** the control unit is designed to create a data stream which comprises the recording data and/or display data, and to provide the data stream via the data interface.

13. Ophthalmological laser therapy system (50), which comprises an optical opening allowing light from a laser source to exit the ophthalmological laser therapy system and which comprises a positioning device (200) according to any of Claims 1 to 12.

14. Method for a positioning device (200) of an ophthalmological laser therapy system (50) comprising an optical opening allowing light from a laser source to exit the ophthalmological laser therapy system, for positioning a patient's eye (170) vis-à-vis the optical opening, the positioning device (200) comprising
- a first recording unit (11, 100, 210) designed to provide first recording data of the eye (170) from a first recording direction,
- a second recording unit (9, 100, 220) designed to provide second recording data of the eye (170) from a second recording direction,
the second recording direction differing from the first recording direction,
- a third recording unit (19) designed to provide third recording data from a third recording direction, the third recording direction differing from the first and second recording directions,
- a displacement unit (240) designed to displace the relative position of the eye (170) vis-à-vis the optical opening on the basis of control commands, and
- a control unit (250),
wherein the method comprises the following steps:
- receiving first, second and third recording data,
- combining the first, second and third recording data by calculation to form control commands and
- transmitting the control commands to the displacement unit (240).

15. Method for a positioning device (200) of an ophthalmological laser therapy system (50) comprising an optical opening allowing light from a laser source to exit the ophthalmological laser therapy system, for positioning a patient's eye (170) vis-à-vis the optical opening, the positioning device (200) comprising
- a first recording unit (11, 100, 210) designed to provide first recording data of the eye (170) from a first recording direction,
- a second recording unit (9, 100, 220) designed to provide second recording data of the eye (170) from a second recording direction,
the second recording direction differing from the first recording direction,
- a third recording unit (19) designed to provide third recording data from a third recording direction, the third recording direction differing from the first and second recording directions,
- a display unit (12, 22, 230) for displaying the first, second and third recording data of the eye (170),
- a displacement unit (240) designed to displace the relative position of the eye (170) vis-à-vis the optical opening on the basis of control commands,
- an input unit (20, 260) designed to enable an input of input data, and
- a control unit (250),
wherein the method comprises the following steps:
- receiving first, second and third recording data,
- displaying the first, second and third recording data on the display unit (12, 22, 230),
- receiving input data,
- combining the input data by calculation to form control commands and
- transmitting the control commands to the displacement unit (240).

## Revendications

1. Dispositif de positionnement (200) pour un système de thérapie laser ophtalmologique (50) qui présente une ouverture optique pour faire sortir la lumière d'une source laser du système de thérapie laser ophtalmologique afin de positionner un œil (170) d'un patient par rapport à l'ouverture optique, le dispositif de positionnement (200) comprenant
- une première unité d'enregistrement (11, 100, 210) qui est réalisée pour fournir des premières données d'enregistrement de l'œil (170) à partir d'une première direction d'enregistrement,
- une deuxième unité d'enregistrement (9, 100, 220) qui est réalisée pour fournir des deuxièmes données d'enregistrement de l'œil (170) à partir d'une deuxième direction d'enregistrement,
dans lequel la deuxième direction d'enregistrement est différente de la première direction d'enregistrement,
- une troisième unité d'enregistrement (19) qui est réalisée pour fournir des troisièmes données d'enregistrement à partir d'une troisième direction d'enregistrement,
dans lequel la troisième direction d'enregistrement est différente de la première et de la deuxième direction d'enregistrement,
- une unité de décalage (240) qui est réalisée pour décaler la position relative de l'œil (170) par rapport à l'ouverture optique sur la base d'instructions de commande, et
- une unité de commande qui est réalisée pour générer des instructions de commande sur la base des premières, deuxièmes et troisièmes données d'enregistrement et pour les fournir à l'unité de décalage (240)

2. Dispositif de positionnement (200) pour un système de thérapie laser ophtalmologique (50) qui présente une ouverture optique pour faire sortir la lumière d'une source laser du système de thérapie laser ophtalmologique afin de positionner un œil (170) d'un patient par rapport à l'ouverture optique, le dispositif de positionnement (200) comprenant
- une première unité d'enregistrement (11, 100, 210) qui est réalisée pour fournir des premières données d'enregistrement de l'œil (170) à partir d'une première direction d'enregistrement,
- une deuxième unité d'enregistrement (9, 100, 220) qui est réalisée pour fournir des deuxièmes données d'enregistrement de l'œil (170) à partir d'une deuxième direction d'enregistrement,
dans lequel la deuxième direction d'enregistrement est différente de la première direction d'enregistrement,
- une troisième unité d'enregistrement (19) qui est réalisée pour fournir des troisièmes données d'enregistrement à partir d'une troisième direction d'enregistrement,
dans lequel la troisième direction d'enregistrement est différente de la première et de la deuxième direction d'enregistrement,
- une unité de représentation (12, 22, 230) pour représenter les premières, deuxièmes et troisièmes données d'enregistrement de l'œil (170),
- une unité de décalage (240) qui est réalisée pour décaler la position relative de l'œil (170) par rapport à l'ouverture optique sur la base d'instructions de commande,
- une unité d'entrée (20, 260) qui est réalisée pour permettre d'entrer des données d'entrée, et
- une unité de commande (250) qui est réalisée pour générer des instructions de commande sur la base des données d'entrée ou pour générer des instructions de commande sur la base des données d'entrée et sur la base des données d'enregistrement et pour fournir les instructions de commande à l'unité de décalage (240)

3. Dispositif de positionnement (200) selon l'une quelconque des revendications 1 ou 2, **caractérisé en ce que** la première unité d'enregistrement (11, 100, 210) comprend une première optique d'enregistrement et la deuxième unité d'enregistrement (9, 100, 220) comprend une deuxième optique d'enregistrement, dans lequel au moins une optique d'enregistrement présente côté objet une ouverture numérique inférieure à 0,25, de préférence inférieure à 0,1, de manière particulièrement préférée inférieure à 0,05.

4. Dispositif de positionnement (200) selon l'une quelconque des revendications 1 à 3, dans lequel le système de thérapie laser ophtalmologique (50) présente une optique, **caractérisé en ce que** la première unité d'enregistrement (11, 100, 210) comprend une première optique d'enregistrement, dans lequel une partie de l'optique du système de thérapie laser ophtalmologique (50) et une partie de la première optique d'enregistrement sont identiques.

5. Dispositif de positionnement (200) selon l'une quelconque des revendications 3 ou 4, **caractérisé en ce que** la première optique d'enregistrement est réalisée de telle sorte qu'avec une distance croissante par rapport à la première optique d'enregistrement, un champ de vision d'une région observée est représenté avec une taille de champ de vision constante ou avec une taille de champ de vision supérieure.

6. Dispositif de positionnement (200) selon l'une quelconque des revendications 1 à 5, **caractérisé en ce qu'**une unité d'enregistrement présente dans un plan focal une taille de champ de vision d'au moins 30 mm x 30 mm, de préférence d'au moins 40 mm x 40 mm, de manière particulièrement préférée d'au moins 50 mm x 50 mm.

7. Dispositif de positionnement (200) selon l'une quelconque des revendications 1 à 6, **caractérisé en ce que** la première direction d'enregistrement et la deuxième direction d'enregistrement présentent un angle de 90° ±30° l'une par rapport à l'autre, de préférence de 90° ±10°, de manière particulièrement préférée de 90° ±5°.

8. Dispositif de positionnement (200) selon l'une quelconque des revendications 1 à 7, **caractérisé en ce que** la troisième direction d'enregistrement et la première direction d'enregistrement présentent un angle compris entre 10° et 90° l'une par rapport à l'autre, de préférence entre 20° et 70°, de manière particulièrement préférée entre 30° et 60°.

9. Dispositif de positionnement (200) selon l'une quelconque des revendications 1 à 8, **caractérisé en ce que** l'unité de commande (250) présente une unité de calcul (255) qui est réalisée pour convertir des données d'enregistrement d'une unité d'enregistrement (100, 210, 220) avec un réticule (38) en données de représentation, et **en ce que** l'unité de commande (250) fournit les données de représentation.

10. Dispositif de positionnement (200) selon l'une quelconque des revendications 1 à 9, **caractérisé en ce que** le dispositif de positionnement (200) présente une unité d'éclairage (270) qui est réalisée pour soumettre l'œil (170) à une lumière d'éclairage.

11. Dispositif de positionnement (200) selon l'une quelconque des revendications 1 à 10, **caractérisé en ce que** l'unité de commande (250) présente une unité de calcul (255) qui est réalisée pour effectuer les calculs suivants en utilisant les données d'enregistrement :
- une reconnaissance faciale du patient,
- une reconnaissance d'un œil (170) droit ou gauche du patient,
- une lecture d'un code à barres,
- une détection de mouvements du patient.

12. Dispositif de positionnement (200) selon l'une quelconque des revendications 1 à 11, **caractérisé en ce que** l'unité de commande (255) présente une interface de données, et **en ce que** l'unité de commande est réalisée pour créer un flux de données qui comprend les données d'enregistrement et/ou les données de représentation, et pour fournir le flux de données par l'intermédiaire de l'interface de données.

13. Système de thérapie laser ophtalmologique (50) qui présente une ouverture optique pour faire sortir la lumière d'une source laser du système de thérapie laser ophtalmologique et qui présente un dispositif de positionnement (200) selon l'une quelconque des revendications 1 à 12.

14. Procédé pour un dispositif de positionnement (200) d'un système de thérapie laser ophtalmologique (50) qui présente une ouverture optique pour faire sortir la lumière d'une source laser du système de thérapie laser ophtalmologique afin de positionner un œil (170) d'un patient par rapport à l'ouverture optique, le dispositif de positionnement (200) présentant
- une première unité d'enregistrement (11, 100, 210) qui est réalisée pour fournir des premières données d'enregistrement de l'œil (170) à partir d'une première direction d'enregistrement,
- une deuxième unité d'enregistrement (9, 100, 220) qui est réalisée pour fournir des deuxièmes données d'enregistrement de l'œil (170) à partir d'une deuxième direction d'enregistrement,
dans lequel la deuxième direction d'enregistrement est différente de la première direction d'enregistrement,
- une troisième unité d'enregistrement (19) qui est réalisée pour fournir des troisièmes données d'enregistrement à partir d'une troisième direction d'enregistrement,
dans lequel la troisième direction d'enregistrement est différente de la première et de la deuxième direction d'enregistrement,
- une unité de décalage (240) qui est réalisée pour décaler la position relative de l'œil (170) par rapport à l'ouverture optique sur la base d'instructions de commande, et
- une unité de commande (250),
et le procédé comprenant les étapes consistant à :
- recevoir des premières, deuxièmes et troisièmes données d'enregistrement,
- convertir les premières, deuxièmes et troisièmes données d'enregistrement en instructions de commande, et
- exporter les instructions de commande vers l'unité de décalage (240).

15. Procédé pour un dispositif de positionnement (200) d'un système de thérapie laser ophtalmologique (50) qui présente une ouverture optique pour faire sortir la lumière d'une source laser du système de thérapie laser ophtalmologique afin de positionner un œil (170) d'un patient par rapport à l'ouverture optique, le dispositif de positionnement (200) présentant
- une première unité d'enregistrement (11, 100, 210) qui est réalisée pour fournir des premières données d'enregistrement de l'œil (170) à partir d'une première direction d'enregistrement,
- une deuxième unité d'enregistrement (9, 100, 220) qui est réalisée pour fournir des deuxièmes données d'enregistrement de l'œil (170) à partir d'une deuxième direction d'enregistrement,
dans lequel la deuxième direction d'enregistrement est différente de la première direction d'enregistrement,
- une troisième unité d'enregistrement (19) qui est réalisée pour fournir des troisièmes données d'enregistrement à partir d'une troisième direction d'enregistrement,
dans lequel la troisième direction d'enregistrement est différente de la première et de la deuxième direction d'enregistrement,
- une unité de représentation (12, 22, 230) pour représenter les premières, deuxièmes et troisièmes données d'enregistrement de l'œil (170),
- une unité de décalage (240) qui est réalisée pour décaler la position relative de l'œil (170) par rapport à l'ouverture optique sur la base d'instructions de commande,
- une unité d'entrée (20, 260) qui est réalisée pour permettre d'entrer des données d'entrée, et
- une unité de commande (250),
et le procédé comprenant les étapes consistant à :
- recevoir des premières, deuxièmes et troisièmes données d'enregistrement,
- représenter les premières, deuxièmes et troisièmes données d'enregistrement sur l'unité de représentation (12, 22, 230),
- recevoir des données d'entrée,
- convertir les données d'entrée en instructions de commande, et
- exporter les instructions de commande vers l'unité de décalage (240).
